# EUROPÄISCHE PATENTANMELDUNG

(11) **EP 1 657 677 A1**
(43) Veröffentlichungstag der Anmeldung: **17.05.2006**
(21) Anmeldenummer: 04024685.2
(22) Anmeldetag: 15.10.2004
(51) Int. Cl.: G06T 7/00, A61B 5/053, A61M 5/172, A61B 5/04, A61N 1/05, A61B 5/0478, A61B 19/00

(54) **Verfahren und Vorrichtung zur Bestimmung des Ortes einer elektrischen Aktivität von Nervenzellen**

(71) Anmelder: BrainLAB AG, 85551 Kirchheim/Heimstetten (DE)
(72) Erfinder: Hartlep, Dr. Andreas, 83629 Naring (DE); Pedain, Dr. Christoph, 81667 München (DE)
(74) Vertreter: Gassenhuber, Andreas

(57) **Zusammenfassung**

Die Erfindung betrifft ein Verfahren zur Bestimmung des Ortes einer elektrischen Aktivität oder Hyperaktivität von Nervenzellen in einer anatomischen Struktur eines Körpers, wobei ein Modell der elektrischen Leitfähigkeit der anatomischen Struktur erstellt wird, mit mindestens einer Elektrode elektrische Impulse erfasst werden und aus den erfassten elektrischen Impulsen unter Verwendung des Modells zur Beschreibung der elektrischen Leitfähigkeit der anatomischen Struktur bestimmt wird, wo der oder die Orte der elektrischen Aktivität in der anatomischen Struktur sind, sowie eine Vorrichtung zur Bestimmung des Ortes einer elektrischen Aktivität von Nervenzellen in einem Körper oder in Körpergewebe mit einer Datenerfassungseinheit (MRI) zur patientenspezifischen Erfassung von Parametern zur Beschreibung der elektrischen Leitfähigkeit der Körperstruktur, einer Recheneinheit (PC), welche mit der Datenerfassungseinheit (MRI) verbunden ist und von dieser die patientenspezifischen Daten erhält, einer Datenbank (DATA), welche mit der Recheneinheit (PC) verbunden ist und in welcher Daten zur Beschreibung von elektrischen Eigenschaften eines bestimmten Gewebetyps oder einer bestimmten Körperstruktur, Daten zur Beschreibung von Mechanismen zur Ausbreitung von elektrischen Signalen und/oder Daten zur Beschreibung der Auswirkung einer Substanz auf die elektrische Leitfähigkeit der Körperstruktur oder von Gewebe abgelegt sind, welche von der Recheneinheit (PC) abgerufen oder patientenspezifisch abgespeichert werden können und mit mindestens einer Elektrode, welche mit der Recheneinheit (PC) verbunden ist und elektrische Signale erfassen kann, so dass die Recheneinheit die erfassten elektrischen Signale unter Verwendung eines erstellten Modells der elektrischen Leitfähigkeit des Körpers oder eines Teiles davon einem oder mehreren Orten oder Bereichen des Körpers zuordnen kann.

## Beschreibung

Die vorliegende Erfindung bezieht sich auf ein Verfahren und eine Vorrichtung zur Bestimmung des Ortes oder Bereiches der elektrischen Aktivität oder Hyperaktivität von Nervenzellen, insbesondere zur Erfassung des Ortes von elektrischen Aktivitäten im Gehirn.

Aus der US 5,735,814 ist ein Verfahren zur Behandlung von neurodegenerativen Erkrankungen bekannt, wobei ein Sensor in das Gehirn eines Patienten implantiert wird und die von Sensorelektroden erfassten elektrischen Potentiale verwendet werden, um die Verabreichung einer Substanz in das Gehirn zu regeln.

Es ist eine Aufgabe der vorliegenden Erfindung ein Verfahren und eine Vorrichtung vorzuschlagen, welche eine exaktere Analyse der elektrischen Aktivität von Nervenzellen insbesondere im Gehirn ermöglichen.

Diese Aufgabe wird durch das Verfahren und die Vorrichtung wie in den unabhängigen Ansprüchen definiert gelöst. Vorteilhafte Ausführungsformen ergeben sich aus den Unteransprüchen.

Bei dem erfindungsgemäßen Verfahren zur Bestimmung des Ortes oder des Bereiches einer elektrischen Aktivität oder Hyperaktivität von Nervenzellen eines Körpers werden mittels mindestens einer und bevorzugt unter Verwendung von 2, 3, 4 oder mehreren Elektroden, welche in den Körper und insbesondere in eine bestimmte anatomische Struktur, wie z. B. das Gehirn oder das Rückenmark implantiert oder an den Körper oder die Struktur angelegt sein können, elektrische Signale erfasst, welche von einer oder mehreren Zellen und insbesondere von bestimmten Aktivitätsbereichen des Gehirns ausgesendet sein können. Die Elektroden können so ausgebildet sein und so positioniert werden wie in der US 5,735,814 beschrieben, deren diesbezügliche Lehre in diese Anmeldung aufgenommen wird. Erfindungsgemäß wird zur Bestimmung des Ortes oder Bereiches der elektrischen Aktivität berücksichtigt, dass die elektrische Aktivität nicht zwangsläufig am Erfassungsort eines Sensors oder einer Elektrode vorliegen muss, sondern dass erfasste elektrische Signale oder Felder auch von Nervenzellen oder Aktivitätszentren stammen können, welche entfernt von dem tatsächlichen Erfassungsort vorliegen können. Hierzu wird ein Modell der elektrischen oder spezifischen elektrischen Leitfähigkeit der untersuchten anatomischen Struktur, insbesondere ein Modell zur Beschreibung oder Simulation der elektrischen Leitfähigkeit oder des elektrischen Widerstandes des Gehirnes eines Patienten erstellt, um unter Verwendung dieses Modells in Verbindung mit den an einer oder mehreren Elektroden erfassten elektrischen Signalen oder Impulsen den oder die Orte einer elektrischen Aktivität oder Hyperaktivität bestimmen zu können. Somit ist es möglich nicht nur die elektrischen Signale in der unmittelbaren Umgebung von Elektroden oder Sensoren zu messen bzw. gemessene elektrische Signale der unmittelbaren Umgebung der Sensoren oder Elektroden zuzuordnen, sondern es kann unter Verwendung des erstellten Modells der elektrischen Leitfähigkeit bzw. des Widerstands oder mit einer Simulation der Leitfähigkeit z. B. der Gehirnsubstanz errechnet oder simuliert werden, wie sich elektrische Signale ausgehend von einem Aktivitätszentrum ausgebreitet haben, bis sie von den jeweiligen Elektroden oder Sensoren erfasst wurden, so dass ein oder mehrere Orte oder Bereiche elektrischer Aktivitäten, wie z. B. ein epileptischer Fokus im Gehirn, durch absolute oder relative Orts- oder Bereichsangaben bestimmt werden kann, ohne dass an diesen Orten eine Elektrode positioniert sein muss. Somit kann mit einem Modell der elektrischen Leitfähigkeit oder des elektrischen Widerstandes der untersuchten Körperstruktur die Bestimmung des Ortes einer elektrischen Aktivität von Gehirnzellen präziser durchgeführt werden.

Allgemein können zur Bestimmung des Modells der allgemeinen oder spezifischen elektrischen Leitfähigkeit oder des Widerstandes patientenspezifische Daten verwendet werden, wie z. B. MR-DT (Kernspinresonanz-Degrees Twaddle) Bilder, mit welchen z. B. die Dichte oder Ionenkonzentration einer Flüssigkeit, insbesondere der Gehirnflüssigkeit, ermittelt werden kann, um ein patientenspezifisches individuelles dreidimensionales Modell der anatomischen und/oder physiologischen Eigenschaften, wie z. B. der elektrischen Leitfähigkeit des Gehirns, zu erstellen. Ebenso können außer MRI auch Ultraschall, Computertomographie (CT), PET, SPECT-Aufnahmen oder andere Mess- oder Datenerfassungsverfahren, wie z. B. Biopsie, verwendet werden, um allgemeine oder elektrische Gehirneigenschaften wie z. B. die anatomische Struktur des Gehirns oder die Verteilung und Leitfähigkeit von Flüssigkeiten im Gehirn zu ermitteln. Weiterhin können auch sogenannte generische Modelle verwendet werden, welche z. B. übliche elektrische Parameter, wie z.B. den spezifischen elektrischen Widerstand von Nervenzellen oder anderen anatomischen Strukturen beschreiben und welche z. B. durch an einem Patienten durchgeführte z.B. bildgebende Messverfahren modifiziert wurden, um ein patientenspezifisches dreidimensionales Modell zur Beschreibung der elektrischen Leitfähigkeit der untersuchten Körperstruktur zu erstellen. Somit werden Körper- oder Gewebeeigenschaften allgemein oder individuell ermittelt, welche für die Ausbreitung elektrischer Signale relevant sind, also insbesondere spezifische Leitfähigkeiten oder Widerstände bestimmt, berechnet oder simuliert, welche sich auf die Ausbreitung eines elektrischen Signals in der betreffenden Körperstruktur auswirken können.

Werden z. B. mittels eines Sensors, an welchem zwei oder mehr Elektroden an unterschiedlichen Positionen des Sensors angebracht sein können, elektrische Signale wie z.B. Potentiale aus der Umgebung des Sensors erfasst, so kann unter Verwendung des erfindungsgemäß ermittelten Modells zur Ausbreitung von elektrischen Signalen in einem Körper oder einer anatomischen Struktur bestimmt werden, wo beispielsweise eine Hyperaktivität von Nervenzellen vorliegt, was z. B. auf einen epileptischen Anfall hindeuten kann. Wird z. B. festgestellt, dass elektrische Aktivitäten vorliegen, welche die Gesundheit oder den Zustand eines Patienten beeinflussen können, so können über in den Körper und insbesondere in das Gehirn eingebrachte Elektroden, welche auch mit den Messelektroden identisch sein können, auch elektrische Signale abgegeben werden, um mögliche Auswirkungen der erfassten elektrischen Aktivitäten zu unterdrücken oder abzuschwächen, so dass beispielsweise ein epileptischer Anfall bereits in der Entstehung erkannt und unterdrückt werden kann. Zur Bestimmung der Art, insbesondere der Impulsfolge und der Energie der durch in den Körper eingebrachten Elektroden abgegebenen Signale, kann auch das erfindungsgemäß ermittelte Modell zur Bestimmung der Ausbreitungsfähigkeit von elektrischen Signalen, also z. B. ein Modell zur dreidimensionalen Beschreibung der elektrischen Leitfähigkeit, verwendet werden, so dass z. B. mittels einer, zwei, drei oder mehr Elektroden verabreichte elektrische Signale so abgegeben werden können, dass in einem gewünschten Bereich des Körpers oder Gehirns z. B. eine Unterdrückung oder Abschwächung einer Hyperaktivität von Gehirnzellen oder Neuronen bewirkt werden kann.

Gemäß einer weiteren Ausführungsform können die erfindungsgemäß gewonnenen Informationen zur Bestimmung des oder der Orte einer elektrischen Aktivität auch verwendet werden, um beispielsweise eine Flüssigkeit oder Substanz in den Körper oder in das Gehirn einzubringen, um z. B. die physiologischen oder elektrischen Eigenschaften des Gehirns zu verändern. Bezüglich des Einbringens von Substanzen in einen Körper und insbesondere in das Gehirn mittels eines Katheters und insbesondere unter Verwendung einer implantierbaren Pumpe, wobei elektrische Signale zur Steuerung der Abgabe der Substanz verwendet werden, wird auf die Lehre der US 5,735,814 verwiesen, welche diesbezüglich in diese Anmeldung aufgenommen wird.

Weiterhin kann die Wirkung der Verabreichung einer oder mehrerer Substanzen auf die elektrische Leitfähigkeit oder den elektrischen Widerstand der betreffenden Körperstruktur simuliert werden, um bei der weiteren Behandlung und insbesondere bei der Berechnung eines Modells der dreidimensionalen elektrischen Leitfähigkeit berücksichtigt zu werden.

Der oder die mit dem oben beschriebenen Verfahren ermittelten Daten zur Bestimmung des Ortes der Bereiche einer elektrischen Aktivität oder Hyperaktivität können an ein bekanntes Navigationssystem übergeben werden, um z. B. eine oder mehrere Elektroden, Katheter oder Pumpen an bestimmten Bereichen positionieren zu können, welche z. B. besonders vorteilhaft sind, um elektrische Signale oder Substanzen an erkrankte Bereiche des Körpers und insbesondere an Bereiche mit hyperaktiven Neuronen abzugeben.

Weiterhin bezieht sich die Erfindung auf ein Computerprogramm, welches, wenn es in einen Computer geladen ist oder auf einem Computer läuft, ein wie oben beschriebenes Verfahren ausführen kann. Ebenso bezieht sich die Erfindung auf ein Programmspeichermedium oder ein Computerprogrammprodukt mit einem solchen Programm.

Eine erfindungsgemäße Vorrichtung zum Bestimmen des Ortes einer elektrischen Aktivität von Nervenzellen weist eine Dateneingabevorrichtung auf, welche z. B. mit einer Datenerfassungseinheit, wie z. B. einem Kernspintomographen oder Computertomographen, verbunden werden kann oder eine solche Einheit ist. Die eingegebenen oder erfassten generischen oder patientenspezifischen Daten werden einer Recheneinheit bzw. einem Computer zugeführt, welcher in der Lage ist, die eingegebenen Daten, wie z. B. Bilddaten, auszuwerten und welcher weiterhin z. B. aus einer Datenbank anatomische Daten z. B. bezüglich der elektrischen Leitfähigkeit, des Grades der elektrischen Empfindlichkeit oder elektrischen Absorption oder anatomische Informationen z. B. zur Verteilung der weißen und grauen Substanz (white/grey matter) erhält. Die Recheneinheit kann z. B. einen herkömmlichen Computer und/oder ein neuronales Netzwerk und/oder eine Fuzzy-Logik enthalten, um aus einem Datensatz, wie z. B. aus Messergebnissen einer Datenerfassungseinheit zur individuellen Untersuchung eines Körpers oder einen Gewebestruktur, patientenspezifische Parameter, wie z. B. die elektrische Leitfähigkeit des Gewebes oder die Verteilung einer Flüssigkeit in dem Gewebe zu ermitteln und insbesondere ein Modell zur Beschreibung der elektrischen Leitfähigkeit einer Körperstruktur zu erstellen. Weiterhin kann die Recheneinheit vorzugsweise die Verteilung eines Fluids, die Konzentration eines Fluids oder die chemische Beschaffenheit eines oder mehrerer Fluide in der betreffenden Körperregion, wie z. B. im Gehirn, simulieren unter Verwendung der Informationen, welche generisch oder patientenspezifisch erhalten wurden, wobei ergänzend allgemeine biologische oder anatomische Parameter oder Funktionen als generische Daten aus einer Datenbank hinzugezogen werden können. Weiterhin ist die Recheneinheit mit mindestens einer Elektrode oder einem Sensor verbunden, mit welcher elektrische Impulse oder Potentiale z.B. des Gehirns erfasst werden können, um unter Verwendung des erstellten dreidimensionalen Modells zur Beschreibung der spezifischen elektrischen Leitfähigkeit oder des Widerstandes zu ermitteln wo ein oder mehrere Orte sind, von welchen die elektrische Aktivität oder Hyperaktivität ausgegangen ist. Insbesondere kann unter Verwendung des erfindungsgemäß erstellten Modells zur Beschreibung der elektrischen Leitfähigkeit einer anatomischen Struktur oder des Gehirns aus der Intensität und/oder Art der mittels einer Elektrode erfassten elektrischen Signale oder Potentiale berechnet werden, wie weit das Aktivitätszentrum, welches diese elektrischen Signale ausgesendet hat, von der betreffenden Elektrode entfernt ist. Werden zwei, drei oder mehrere Elektroden verwendet, so können unter Verwendung des anatomischen Modells zur Beschreibung der elektrischen Leitfähigkeit relativ genau die Orte eines oder mehrerer Bereiche in dem Körper bestimmt werden, welche die von der oder den Elektroden erfassten elektrischen Signale ausgesandt haben.

Vorzugsweise weist die erfindungsgemäße Vorrichtung Elektroden zur Abgabe von Signalen und/oder einen oder mehrere Katheter und/oder Pumpen zur dosierten Abgabe einer Substanz und/oder ein Navigationssystem auf, mit welchem die Elektroden, Katheter und Pumpen positioniert und z.B. die Durchführung und/oder der Erfolg einer Behandlung der erfindungsgemäß bestimmten Bereiche hyperaktiver Nervenzellen durch Zufuhr einer Substanz oder Anlegen von elektrischen Potentialen überwacht werden können, wobei z. B. im Falle der Abweichung eines tatsächlichen Verlaufs einer Behandlung von einem geplanten oder simulierten Verlauf der Behandlung z. B. zur Unterdrückung einer Hyperaktivität von Nervenzellen eine oder mehrere Behandlungsparameter, wie z. B. über Elektroden verabreichte elektrische Impulse, z. B. hinsichtlich ihrer Intensität oder Impulsformen oder Parameter zur Verabreichung von Substanzen zur Beeinflussung der elektrischen Leitfähigkeit modifiziert werden können.

Die Erfindung wird nachfolgend anhand eines Ausführungsbeispiels beschrieben. Es zeigen:
- Figur 1: ein Ablaufdiagramm einer Ausführungsform des erfindungsgemäßen Verfahrens; und
- Figur 2: eine erfindungsgemäße Vorrichtung.

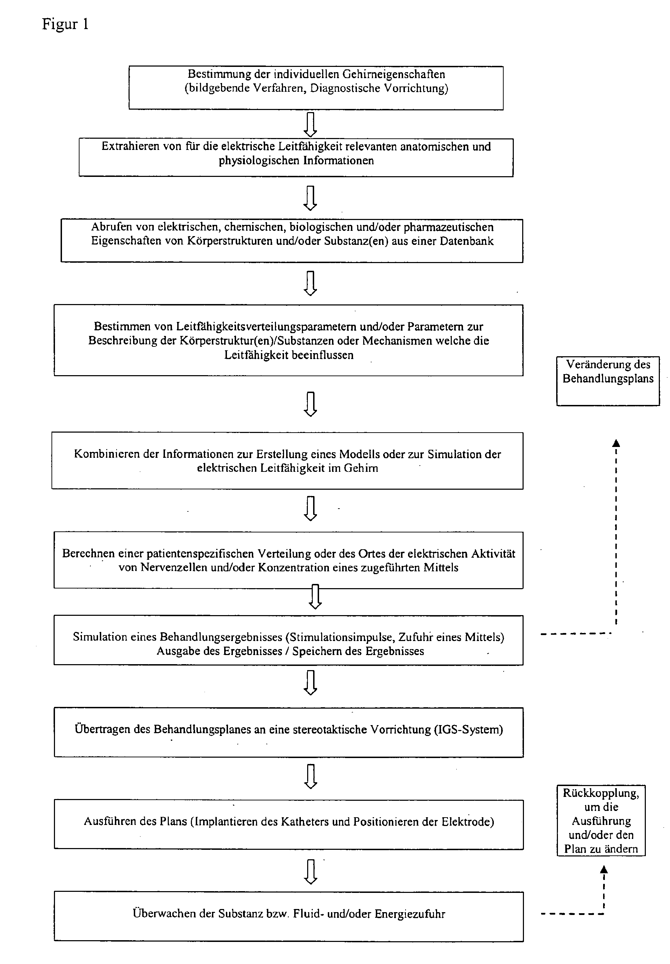

Figur 1 zeigt einen Ablaufplan für eine Ausführungsform des erfindungsgemäßen Verfahrens zur Bestimmung des Bereichs einer elektrischen Hyperaktivität, wobei in einem ersten Schritt patientenspezifisch die individuellen Gehirneigenschaften oder Gehirnparameter bestimmt werden, wozu zum Beispiel bekannte bildgebende Verfahren, insbesondere Kernspintomografie, Ultraschall, Computertomografie, PET, SPECT, Biopsie oder andere bekannte Verfahren verwendet werden können.

Die erfassten Daten zur Beschreibung von Eigenschaften des Gehirns werden anschließend ausgewertet, um relevante anatomische und/oder physiologische und/oder funktionelle Informationen zu erhalten. Hierzu können zum Beispiel ein generisches Modell oder auch bekannte mathematische Modelle zur Ermittlung von spezifischen für die Verteilung einer Substanz oder für die Absorption oder Weiterleitung von Energie relevante Eigenschaften aus den erfassten Daten verwendet werden. Insbesondere können zum Beispiel die (spezifische) elektrische Leitfähigkeit, das thermische Diffusionsvermögen, der Verlauf von Nervenbahnen, die Dichteverteilung, die Elastizität, der Flüssigkeitsgehalt, der Blutgehalt, Angaben zum Blutfluss, die vaskulare Permeabilität oder andere Informationen zur Beschreibung von Eigenschaften oder Strukturen des Gehirns verwendet werden. Werden zum Beispiel aus einem generischen Modell bekannte Muster oder allgemeine Informationen verwendet, so können Eigenschaften des Gehirns für bestimmte Volumen oder Gehirnbereiche abgeschätzt werden, wenn diese nicht direkt durch Messung ermittelt wurden oder werden können.

Die durch eine Messung und/oder ein generisches Modell oder aus einer Datenbank ermittelten Informationen, welche für das erfindungsgemäße Verfahren relevant sein können, können eine oder mehrere der folgenden Mechanismen oder Eigenschaften sein:
- Transportmechanismus im Körper (zum Beispiel für Körperflüssigkeiten oder extern verabreichte Flüssigkeiten oder Substanzen)
- Abfluss oder Verteilung einer Flüssigkeit in dem Körper oder Gewebe (abhängig unter anderem von der Permeabilität oder von physiologischen Eigenschaften, wie beispielsweise der Blut-Hirn-Schranke)
- Hydraulische und/oder chemische und/oder biologische Eigenschaften an Körperstrukturgrenzen (Blut-Hirn-Schranke, Sulcii)
- Diffusion, welche sich auf den Transportmechanismus einer Substanz im Gewebe auswirkt
- elektrische Leitfähigkeit
- Grad der Energie-Sensitivität oder Energie-Empfindlichkeit
- Grad der Energie-Absorption
- Anatomische Information (weiße/graue Substanz)

Wurde unter Verwendung von einer oder mehrerer der oben beschriebenen Eigenschaften oder Mechanismen ein Modell des Gehirns oder der zu behandelnden Körperregion zur Beschreibung der elektrischen Leitfähigkeit erstellt, so kann eine lokale Verteilung oder die Ausbreitung und Abschwächung von elektrischen Signalen im Gehirn simuliert werden, um z.B. ausgehend von einer Messelektrode das elektrische Signal simuliert im Modell zu seinem Entstehungsort zurück zu verfolgen. Zur Simulation können elektrische aber auch allgemeine chemische (Ionenkonzentration), biologische und/oder pharmazeutische Eigenschaften der Körperstruktur oder einer verabreichten Substanz zum Beispiel aus einer Datenbank gewonnen werden, wobei ergänzend eine oder mehrere der folgenden Informationen oder Eigenschaften zur Simulation der Leitfähigkeitsverteilung oder des Ausbreitungsmechanismus von elektrischen Signalen herangezogen werden können:
- Eine Information bezüglich der Verabreichungsparameter einer Substanz (wie zum Beispiel die lokale Verabreichung in Gewebe oder die systemische Verabreichung; die Dauer der Verabreichung oder Infusion, die Auswirkung des Stoffwechsels (Metabolisation) auf die aktive Substanz oder den Trägermechanismus oder die Trägersubstanz, die Anordnung der Zuführvorrichtungen (wie zum Beispiel Katheter und Pumpen)
- Gemessene oder zugewiesene bzw. bestimmte elektrische Eigenschaften des anatomischen Bereiches oder -gewebes
- Pharmazeutische Eigenschaften des jeweiligen Mittels (wie zum Beispiel wärmeempfindliche Liposome, Energie oder Partikel emittierende Flüssigkeiten, Strahlungs-Sensitizer, Toxine, ...)
- Anordnung und/oder Eigenschaften der Elektrode(n)
- mathematisches Modell, welches die oben erwähnten Informationen verwendet und mit dem sich eine Flüssigkeitsverteilung und/oder Dosis berechnen lässt

Weiterhin werden Parameter zur elektrischen Energiezufuhr und Informationen bezüglich der Vorrichtung oder Mechanismen, mit welchen die elektrische Energie zugeführt wird, verwendet, um die Zufuhr von elektrischer Energie oder Signalen in das Zielgewebe zu simulieren. Zur Simulation der Energiezufuhr und insbesondere zur Berechnung des anzulegenden Signals (Stärke, Signalform) können eine oder mehrere der folgenden Informationen verwendet werden:
- gemessene oder zugeordnete Eigenschaften des Zielbereiches
- eine Information zur Anordnung der Elektroden oder Konzentration von Substanzen, Objekten oder Flüssigkeiten, welche sich auf die Energiesensitivitäts- und/oder die Absorptionsparameter des Zielbereiches auswirken
- eine Lageinformation der elektrischen Energie emittierenden Objekte oder Flüssigkeiten
- Emissionseigenschaften der Objekte oder Flüssigkeiten
- Zeitparameter zur Objekt- oder Flüssigkeitsanordnung oder -Verteilung
- Energieverabreichungsparameter aus Quellen, welche außerhalb des Zielbereiches angeordnet sind
- ein mathematisches Modell, welches obige Informationen verwendet und eine lokale Verteilung der Energiezufuhr bestimmen kann.

Erfindungsgemäß werden die oben beschriebenen Simulationsergebnisse kombiniert, das heißt, es wird unter Berücksichtigung des Leitfähigkeitsmodells oder der simulierten Verteilung einer oder mehrerer Substanzen oder Flüssigkeiten in einem Gewebe oder einer Körperstruktur ermittelt, wie sich die simulierte Verabreichung von elektrischer Energie gemäß einem Behandlungsplan zur zum Beispiel unterschiedlich dosierten Zufuhr von Energie mit verschiedenen Elektroden in verschiedenen Körperbereichen auswirkt. Dabei kann zum Beispiel bestimmt werden, ob mit den zur Simulation verwendeten Daten des Behandlungsplanes, wie zum Beispiel dem Ort einer Elektrode oder eines Katheters zum Einbringen einer Substanz und einer errechneten Signalform oder Abgabekennlinie der Substanz zum Beispiel zur Beschreibung des zeitlichen Verlaufs der abgegebenen Menge oder des Druckes, mit welcher die Substanz in das Gewebe eingebracht wird und unter Berücksichtigung der Mechanismen zur Zufuhr von Energie, zum Beispiel Hyperaktivitätszentren in einer ausreichenden Menge erreicht und/oder beeinflusst werden können.

Die berechnete patientenspezifische Anordnung der Elektroden, die anzulegenden Signalformen, die Konzentration der eingebrachten Substanz und/oder die Verteilung der Substanz bzw. der Flüssigkeit und/oder die Energieverteilung werden erfindungsgemäß berechnet, wobei das Ergebnis ausgegeben und gespeichert werden kann.

Es ist möglich, die Reaktion des Zielbereiches, also z. B. des Hyperaktivitätszentrums, über die Zeit zu simulieren, wobei auch die Parameter zur Beschreibung des Gewebeverhaltens über die Zeit verändert werden können, um das dynamische Verhalten des Zielbereiches zu beschreiben, so dass zum Beispiel auch durch die Behandlung auftretende Effekte oder Auswirkungen auf das Gewebe, zum Beispiel durch die zugeführten elektrischen Signale, die verabreichte Substanz oder die zugeführte Energie für die Planung der Behandlung berücksichtigt werden können.

Ausgegeben werden kann zum Beispiel ein von einer Recheneinheit unter Verwendung des oben beschriebenen Verfahrens ermittelter Datensatz zum Beispiel in grafischer Darstellung, um eine oder mehrere der folgenden Informationen anzuzeigen:
- Verteilung der Quellen der elektrischen Signale, der verabreichten Substanzen und/oder Trägersubstanzen der Substanzen oder Medikamente
- lokal variierende Konzentration der Substanzen und/oder Trägersubstanzen, welche die elektrischen Eigenschaften z. B. des Gewebes beeinflussen
- Energieverteilung im Gewebe bei leitenden oder energieemittierenden Substanzen
- Energieverteilung einer externen Energiequelle
- Energieverteilung unter Berücksichtigung der Wirkung der verabreichten elektrischen Energie, Flüssigkeiten oder Substanzen, welche die Energieabsorption und/oder Energieempfindlichkeit des Zielbereiches verändern (Wirkungsverteilung).

Wird nach der Simulation festgestellt, dass ein gewünschtes oder vorgegebenes Behandlungsergebnis nicht oder nicht vollständig erreicht wird, so kann das Verfahren iterativ durchgeführt werden, wobei Behandlungsparameter, wie zum Beispiel die Dosierung oder Verabreichungsmechanismen der extern zugeführten elektrischen Energie (Ort der Elektroden; Dauer, Stärke, zeitlicher Verlauf der elektrischen Signale), Substanzen oder Flüssigkeiten oder die Mechanismen zur Zufuhr von Energie verändert werden und eine erneute Simulation mit den veränderten Ausgangsparametern durchgeführt wird, bis ein zufriedenstellendes Simulationsergebnis erhalten wird.

Ist das Simulationsergebnis zufriedenstellend, so kann der Behandlungsplan zum Beispiel an eine stereotaktische Vorrichtung übertragen werden, wie zum Beispiel ein bildunterstütztes Chirurgie (IGS)-System, um den Behandlungsplan auszuführen und um zum Beispiel eine oder mehrere Elektroden und/oder Katheter an den durch die Simulation als günstig ermittelten Stellen zu platzieren.

Ergänzend kann während der Durchführung der Behandlung die Zufuhr der Flüssigkeit bzw. der Substanz und/oder der elektrischen Energie gemessen oder überwacht werden, um dem Anwender eine Rückmeldung zu geben, ob der Behandlungsplan richtig ausgeführt wird oder geändert werden sollte, so dass zum Beispiel Parameter zur Zufuhr der Substanz bzw. einer Flüssigkeit oder zur Zufuhr der elektrischen Energie eingestellt oder abgeändert werden können, um ein besseres Behandlungsergebnis zu erreichen.

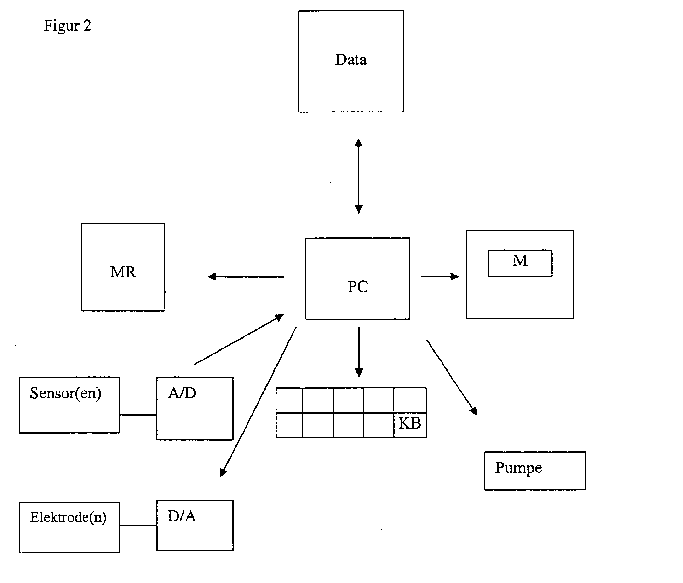

Figur 2 zeigt eine Ausführungsform der erfindungsgemäßen Vorrichtung, wobei ein Kernspinresonanztomograph MRI patentenspezifische Daten erfasst und an eine Recheneinheit PC ausgibt. Die Recheneinheit PC ist mit einer Datenbank DATA verbunden, in welcher ein generisches Modell zur Beschreibung der dreidimensionalen elektrischen Leitfähigkeitsverteilung des von dem Kernspintomographen MRI erfassten Körperbereiches abgelegt ist, wobei die Recheneinheit PC aus den von dem Kernspintomographen MRI und der Datenbank DATA übermittelten Daten ein Modell der Leitfähigkeitsverteilung der Körperstruktur bildet, welches die patientenspezifischen Parameter zur Verteilung oder Ausbreitung von in der Körperstruktur erzeugten oder zugeführten elektrischen Signalen beschreibt.

Über eine Eingabeeinheit, wie zum Beispiel eine Tastatur KB, werden der Recheneinheit PC von einem Benutzer weitere Daten zum Beispiel bezüglich der Art einer zu verabreichenden Substanz oder der Art der zugeführten elektrischen Energie eingegeben, wobei die Recheneinheit PC hierzu aus der Datenbank DATA ergänzende Informationen zum Beispiel bezüglich physikalischer, chemischer oder physiologischer Eigenschaften der Substanz oder der verwendeten Elektroden abrufen kann, um das oben beschriebene Verfahren auszuführen. Die von der Recheneinheit PC ermittelten Simulationsergebnisse können an einem Monitor M grafisch dargestellt werden.

Mit der Recheneinheit PC sind ein oder mehrere Sensoren mittels eines A/D Wandlers verbunden, welche z. B. in das Gehirn eines Patienten eingebracht werden können und erfasste elektrische Signale an die Recheneinheit PC abgeben, um erfindungsgemäß den Ort der Entstehung der elektrischen Signale zu bestimmen. Zur Behandlung von Funktionsstörungen des Gehirns können ein oder mehrere Elektroden, welche auch mit den beschriebenen Sensoren identisch sein können, über einen D/A Wandler mit der Recheneinheit PC verbunden sein, um elektrische Signale zur Therapie von Funktionsstörungen des Gehirns abgeben zu können.

Weiterhin kann mit der Recheneinheit eine wie in der US 5,735,814 beschriebene Pumpe verbunden sein.

## Patentansprüche

1. Verfahren zur Bestimmung des Ortes einer elektrischen Aktivität oder Hyperaktivität von Nervenzellen in einer anatomischen Struktur eines Körpers, wobei ein Modell der elektrischen Leitfähigkeit der anatomischen Struktur erstellt wird, mit mindestens einer Elektrode elektrische Impulse erfasst werden und aus den erfassten elektrischen Impulsen unter Verwendung des Modells zur Beschreibung der elektrischen Leitfähigkeit der anatomischen Struktur bestimmt wird, wo der oder die Orte der elektrischen Aktivität in der anatomischen Struktur sind.

2. Verfahren nach Anspruch 1, wobei patientenspezifische Daten, insbesondere mit einem bildgebenden Verfahren gewonnene Daten, zur Erstellung des Modells zur Beschreibung der elektrischen Leitfähigkeit der anatomischen Struktur verwendet werden.

3. Verfahren nach einem der vorhergehenden Ansprüche, wobei generische Daten zur Erstellung des Modells zur Beschreibung der elektrischen Leitfähigkeit der anatomischen Struktur verwendet werden.

4. Verfahren nach einem der vorhergehenden Ansprüche, wobei über mindestens eine Elektrode elektrische Signale an die anatomische oder Körperstruktur abgegeben werden, deren Intensität und/oder Impulsform unter Verwendung des Modells zur Beschreibung der elektrischen Leitfähigkeit der anatomischen Struktur ermittelt wird.

5. Verfahren nach einem der vorhergehenden Ansprüche, wobei eine Pumpe zur Abgabe einer Substanz in die anatomische Struktur in Abhängigkeit von dem bestimmten Ort der elektrischen Aktivität in der anatomischen Struktur angesteuert wird.

6. Verfahren nach einem der vorhergehenden Ansprüche, wobei die Parameter zur Beschreibung des Ortes einer elektrischen Aktivität in der anatomischen Struktur an ein Navigationssystem übergeben werden, insbesondere um eine oder mehrere Elektroden und/oder Katheter an oder in dem Körper zu positionieren.

7. Computerprogramm, welches, wenn es auf einem Computer läuft oder in einen Computer geladen ist, das Verfahren nach einem der vorhergehenden Ansprüche ausführt.

8. Programmspeichermedium oder Computerprogrammprodukt mit dem Programm nach dem vorhergehenden Anspruch.

9. Vorrichtung zur Bestimmung des Ortes einer elektrischen Aktivität von Nervenzellen in einem Körper oder in Körpergewebe mit einer Datenerfassungseinheit (MRI) zur patientenspezifischen Erfassung von Parametern zur Beschreibung der elektrischen Leitfähigkeit der Körperstruktur, einer Recheneinheit (PC), welche mit der Datenerfassungseinheit (MRI) verbunden ist und von dieser die patientenspezifischen Daten erhält, einer Datenbank (DATA), welche mit der Recheneinheit (PC) verbunden ist und in welcher Daten zur Beschreibung von elektrischen Eigenschaften eines bestimmten Gewebetyps oder einer bestimmten Körperstruktur, Daten zur Beschreibung von Mechanismen zur Ausbreitung von elektrischen Signalen und/oder Daten zur Beschreibung der Auswirkung einer Substanz auf die elektrische Leitfähigkeit der Körperstruktur oder von Gewebe abgelegt sind, welche von der Recheneinheit (PC) abgerufen oder patientenspezifisch abgespeichert werden können und mit mindestens einer Elektrode, welche mit der Recheneinheit (PC) verbunden ist und elektrische Signale erfassen kann, so dass die Recheneinheit die erfassten elektrischen Signale unter Verwendung eines erstellten Modells der elektrischen Leitfähigkeit des Körpers oder eines Teiles davon einem oder mehreren Orten oder Bereichen des Körpers zuordnen kann.

10. Vorrichtung nach dem vorhergehenden Anspruch mit einer Datenausgabeeinheit (M), um das Ergebnis der Zuordnung der Signale zu einem Körperbereich oder den errechneten Ort der Nervenzellenaktivität grafisch darzustellen.

11. Vorrichtung nach einem der beiden vorhergehenden Ansprüche mit mindestens einer mit der Recheneinheit (PC) gekoppelten Elektrode zur Abgabe von elektrischen Signalen an den Körper.

12. Vorrichtung nach einem der drei vorhergehenden Ansprüche mit mindestens einer Pumpe zur dosierten Abgabe einer Substanz an oder in den Körper.

13. Vorrichtung nach einem der vier vorhergehenden Ansprüche mit mindestens einem Navigationssystem, welches mit der Recheneinheit (PC) gekoppelt ist, um Elektroden, Katheter oder Pumpen in oder in der Nähe von Bereichen elektrischer Hyperaktivität positionieren zu können.
